Europäisches Patentamt

(19) European Patent Office

Office européen des brevets

(11) Publication number: **0 156 496**
**B1**

(12) **EUROPEAN PATENT SPECIFICATION**

(45) Date of publication of patent specification: **17.05.89**

(51) Int. Cl.⁴: **A 61 M 1/34**

(21) Application number: **85301121.1**

(22) Date of filing: **20.02.85**

(54) Apparatus for the treatment of plasma.

(30) Priority: **24.02.84 JP 34813/84**
**01.06.84 JP 113495/84**

(43) Date of publication of application:
**02.10.85 Bulletin 85/40**

(45) Publication of the grant of the patent:
**17.05.89 Bulletin 89/20**

(84) Designated Contracting States:
**DE FR GB IT SE**

(56) References cited:
**EP-A-0 044 694**
**EP-A-0 074 610**
**DE-A-3 243 523**
**JP-A-59 008 967**

**TRANSACTIONS OF THE AMERICAN SOCIETY
FOR ARTIFICIAL INTERNAL ORGANS, vol. 29,
Toronto, 28th-30th April 1983, pages 739-743,**

**Lovettsville, Virginia, US; H. VON BAEYER et
al.: "Flow controlled selective plasma
ultrafiltration (SPU) with on line membrane
regeneration by back flush techniques"**

(73) Proprietor: **KURARAY CO., LTD.**
**1621 Sakazu**
**Kurashiki-City Okayama Prefecture 710 (JP)**

(72) Inventor: **Nakano, Akiyoshi**
**2-6-1-205 Fukushima**
**Okayama-City (JP)**
Inventor: **Harada, Yoshimichi**
**2-6-23 Fukushima**
**Okayama-City (JP)**
Inventor: **Kirita, Yasuzo**
**1-3-1- Kita-Midorigaoka**
**Toyonaka-City (JP)**
Inventor: **Miyahara, Tadashi**
**c/o Ranpu-Ryo 1652-1 Sakazu**
**Kurashiki-City (JP)**
Inventor: **Yagiri, Yoshio**
**2-7-32 Hamamachi**
**Kurashiki-City (JP)**
Inventor: **Ueda, Michiko**
**418-1-Ohjima**
**Kurashiki-City (JP)**
Inventor: **Ueda, Yasunori**
**418-1-Ohjima**
**Kurashiki-City (JP)**

(74) Representative: **Topps, Ronald et al**
**D. YOUNG & CO 10 Staple Inn**
**London WC1V 7RD (GB)**

## Description

This invention relates to apparatus for the treatment of plasma. The apparatus is generally capable of operating as a part of a system in which a filtration membrane module thereof is connected to a centrifugal separator. By means of such a system, pathogenic and harmful substances such as immunocompounds, immunoglobulin complexes, and nucleic acids contained in blood can efficiently be removed.

It has recently become apparent that an abnormal increase in the quantity of high molecular weight substances may be a significant cause and/or effect of various diseases such as: autoimmune diseases including rheumatism, SLE, myasthenia gravis, Goodpasture syndrome, and idiopathic thrombocytopenic purpura; abnormal metabolism including multiple myeloma and macrogobulinemis; and hyperviscosity syndrome. Plasmapheresis has come into wide use for the purpose of removing high molecular weight substances. Plasmapheresis is a process for first separating blood from a patient's body into plasma and a corpuscular fraction, removing harmful high molecular weight substances from the separated plasma, and then returning the treated plasma together with the previously separated corpuscular fraction to the patient's body.

Among conventional methods of separating blood into plasma and a corpuscular fraction, it is known to use a membrane separation method using a filtration membrane module, and a centrifugal separation method using a centrifugal separator. Known methods of plasma separation using a membrane module include the following:

(1) a method wherein, after separating blood into plasma and a corpuscular fraction with a plasma separation membrane, a plasma fraction containing harmful substances is removed and a fresh plasma fraction, of a quantity corresponding to that of the removed plasma fraction, is mixed with the corpuscular component and returned to the patient's body;

(2) a method wherein, after separating blood into plasma and a corpuscular fraction with a plasma separation membrane, a plasma fraction containing harmful substances is contacted with an adsorbent so that the harmful substances are adsorbed and removed thereby, and the plasma fraction thus treated is again mixed with the corpuscular component and returned to the patient's body, (US-A-4,013,564; US-A-4,243,532);

(3) a method wherein, after separating blood into plasma and a corpuscular fraction with a plasma separation membrane, the plasma component is further separated into low molecular weight substances containing albumin and high molecular weight substances with a plasma treatment membrane, and then the purified plasma component free of high molecular weight substances, including harmful substances, is mixed with the corpuscular component and returned to the patient's body, (US-A-4,350,594; US-A-4,397,747); and

(4) a method wherein, after separating blood into plasma and a corpuscular fraction with a plasma separation membrane, the plasma fraction is cooled so that high molecular weight substances, including harmful substances, are caused to gel, the gel is removed by a filtration membrane, and only such low molecular weight substances as pass through the filtration membrane are returned to the patient's body after being mixed with the corpuscular fraction, (Artificial Organs, Vol. 4, No. 3, pp 205 - 207, June 1980).

In addition, the following methods are known for plasmapheresis using centrifugal separation methods:

(1) a method wherein, after separating blood into plasma and a corpuscular fraction with a centrifugal separator, the plasma fraction containing harmful substances is removed and fresh plasma, of a quantity corresponding to that of the removed plasma component, is mixed with the corpuscular component and returned to the patient's body; and

(2) a method wherein, after separating blood into plasma and a corpuscular fraction using a centrifugal separator, the plasma fraction is separated into high molecular weight substances and low molecular weight substances with a filtration membrane and purified plasma from which high molecular weight substances alone have been removed is returned to the patient's body together with the corpuscular component, (Japan Laid-open Patent, Nos. 64,058/82 and 8,967/84).

Among these plasmapheresis methods, plasma exchange therapy is associated with the problem of obtaining healthy persons' plasma for transfusion into the patient to be treated and also with secondary effects such as infection by other pathogenic organisms or the contraction of serum sickness occurring with the transfusion of a healthy person's plasma into the patient's body. Therefore the transfusion of the patient's own purified plasma is regarded as preferable. A method of separating blood into plasma and a corpuscular fraction with a centrifugal separator and treating the plasma fraction with a filtration membrane is regarded as quite safe and beneficial in that a fall in separation efficiency for the separation of the blood by the filtration membrane module and risky hemolysis do not occur. However, blood treatment using a system involving a combination of centrifugal separator with filtration membrane is rarely performed these days. The reason for this is considered to be as indicated below.

Centrifugal separators in use today for plasma separation are of two kinds which are different from each other in the way in which they perform, that is:

(1) apparatus for the continuous operation type in which blood is continuously fed into the centrifugal bowl at one side and the corpuscular fraction and plasma are individually and continuously taken out from the bowl at the other side, (e.g. IBM 2,997 and Fenwal CS-3,000); and

(2) apparatus of the batch type in which blood is fed into the bowl at one side and the plasma fraction alone is continuously removed from the bowl at the other side while the corpuscular fraction collects in the bowl until blood flow is stopped immediately after corpuscular fraction sensing means detects corpuscular fraction overflowing the bowl and flowing into the plasma outlet tube, following which the corpuscular fraction collected in the bowl is removed from the blood feed port, (Haemonetics V-50 and Haemonetics PEX).

To connect a hitherto used centrifugal separator with a filtration membrane module, the control circuit of the centrifugal separator must be modified to use closed circuits exclusively for the centrifugal separator or the module and to adapt the control system of the centrifugal separator to control a pump connected to the filtration membrane module. Further, the use of exclusive closed circuits prevents the module from being connected to a centrifugal separator of different mechanism of performance.

H. von Baeyer et al. (Transactions of the American Society for Artificial Internal Organs, 29, Toronto, 28-30 April 1983, pp 739-743, Lovettsville, Virginia, USA: "Flow controlled selective plasma ultrafiltration (SPU) with on line membrane regeneration by back flush techniques") describes plasmapheresis using ultrafiltration apparatus connected to a centrifugal plasma separator. The filtration apparatus described consists of a collection bag for plasma from the centrifugal separator from which plasma is pumped to a first filtration membrane module. Phosphate neutralized saline dilution fluid is simultaneously pumped with the plasma to the filtration module. The condensed fraction from the first filtration module containing high molecular weight substances retained by the membrane is pumped to waste, while the filtrate from the first module is collected in a bag before being pumped to a second filtration membrane module. In the second module, the dilution fluid is retrieved as the filtrate for recycling to the first module, and the condensed plasma fraction from the second module is pumped to a reservoir prior to re-introduction to the patient. The preamble of Claim 1 is based on this disclosure.

We have now found it possible to provide a plasma treatment apparatus having filtration membrane module for treating plasma and which can be connected to any type of centrifugal separator.

We have also found it possible to provide a plasma treatment apparatus which can be controlled independently of the control system in the centrifugal separator and capable of operating as a systematised unit when connected to the centrifugal separator.

According to the present invention there is provided an apparatus for the treatment of plasma of a patient, which is connectable to a centrifugal separator (50) disposed in an extracorporeal circuit so as to separate a plasma component separated from blood by the centrifugal separator into high molecular weight substances and low molecular weight substances containing albumin by means of a filtration membrane module (6) and to return purified plasma freed of high molecular weight substances to the body of the patient, comprising: a plasma feeding circuit (16) for connecting the centrifugal separator (50) disposed in said extracorporeal circuit with the filtration membrane module (6); a plasma reservoir (4) in said circuit; a plasma feed pump (5); a circuit (17) for returning purified plasma containing albumin and freed of high molecular weight substances by said filtration membrane module (6) to the patient's body; a purified plasma reservoir vessel (8) in said circuit (17); a purified plasma delivery pump (7) in the circuit (17) for returning purified plasma to the patient's body, in which pump the fluid flow ratio between plasma introduced to the filtration member (6) and purified plasma delivered therefrom is at a predetermined value and controlled in association with said plasma feed pump (5); and a condensed plasma discharge pump (26) the flow rate of which is set relative to that of the plasma feed pump (5) such that the fluid flow ratio between the plasma component delivered to the filtration member module (6) and high molecular weight substances discharged therefrom to a condensed plasma discharge circuit (27) from the filtration membrane module (6) is at a predetermined value; characterised in that the apparatus includes means (21) for sensing the fluid level in said plasma reservoir (4) whereby the fluid flow rate in the plasma feed pump (5) can be adjusted and controlled in association with said fluid level sensing means (21) so that the fluid level is within a predetermined range; a supplementary fluid circuit (13) connected to the upstream side of the purified plasma delivery pump (7); and a circuit (24) for bypassing the purified plasma delivery pump (7) when flow of purified plasma is interrupted, causing the purified plasma delivery pump (7) to be stopped.

Preferred embodiments of the invention and combinations thereof with different types of centrifugal separators are referred to in the dependent claims.

Embodiments of the invention will now be described, by way of non-limiting example, with reference to the accompanying drawings, in which:-

Figure 1 is a schematic circuit diagram of a system comprising a continuous operation type centrifugal separator and an apparatus for plasma treatment according to one embodiment of the present invention connected together;

Figure 2 is an electrical circuit diagram of a fluid level sensing means of a plasma reservoir bag;

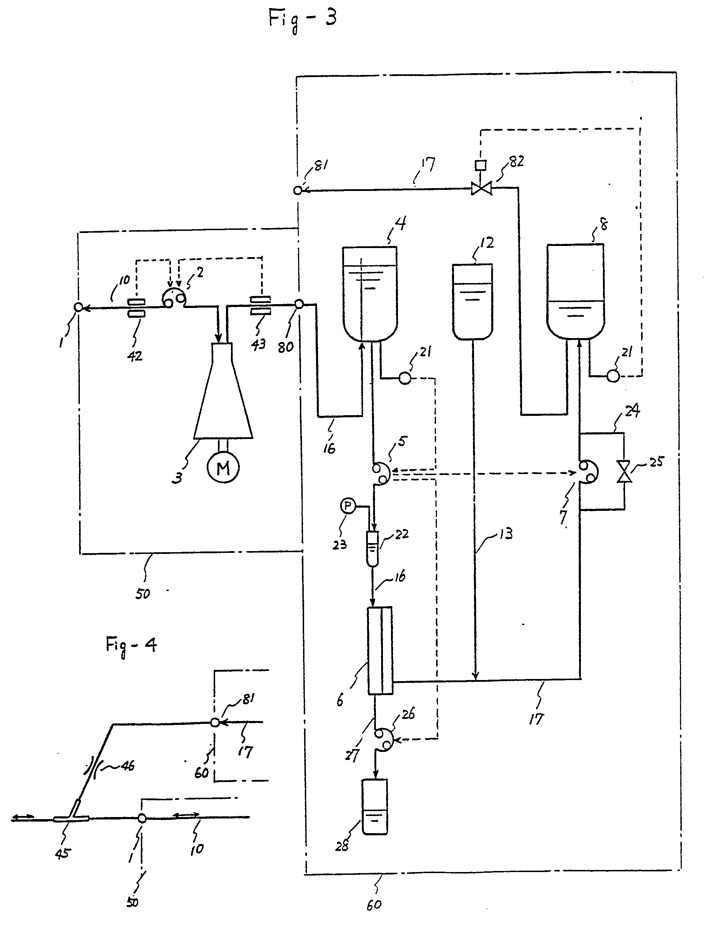

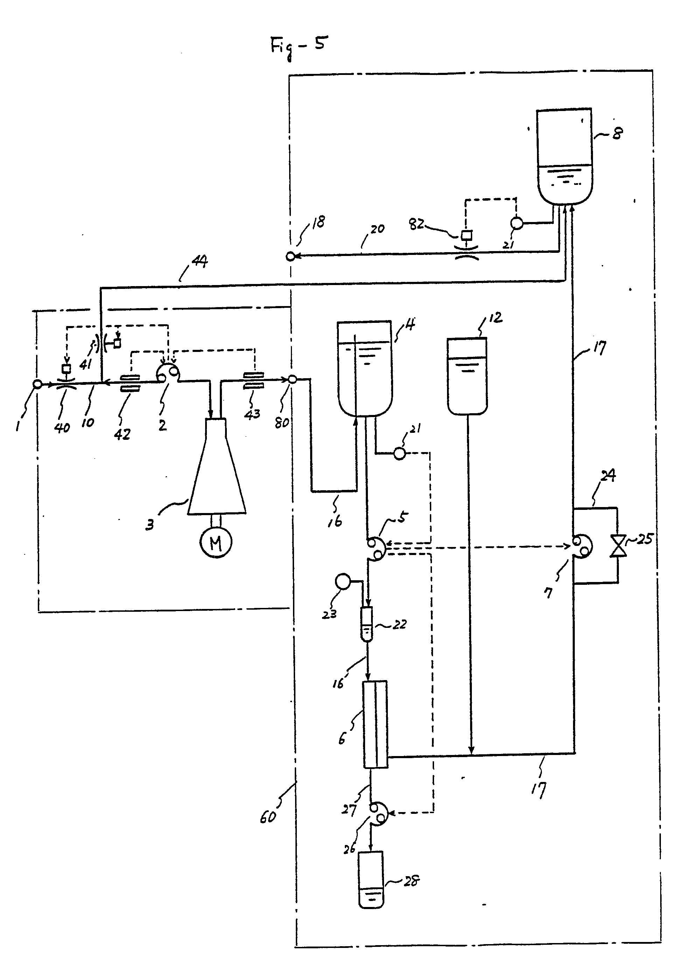

Figures 3 to 5 are schematic circuit diagrams of systems comprising a batch type centrifugal separator and a plasma treatment apparatus according to embodiments of the invention connected together, Figures 3 and 5 showing embodiments of apparatus of the present invention used as a part of a system for a "two-arm" method by which a corpuscular fraction and purified plasma are separately returned to the human body, while Figure 4 represents an embodiment of apparatus of the present invention used as a part of a system for a "single-arm" method by which a corpuscular fraction and purified plasma are mixed together and then returned to the human body; and

Figure 6 is a perspective view of an embodiment of apparatus for plasma treatment according to the present invention.

Plasma treatment apparatus according to embodiments of the invention may be connected to a centrifugal separator so as to provide a system for separating a plasma fraction obtained from blood with a centrifugal separator into high molecular weight substances and low molecular weight substances containing albumin by means of a filtration membrane module and returning a purified plasma component freed of high molecular weight substances to a patient's body. Centrifugal separators which may be connected to plasma treatment apparatus of this invention, include any of the above mentioned continuous operation and batch type separators.

Filtration membrane modules which may be used for plasma separation in plasma treatment apparatus of the invention, applicable include modules incorporating flat membranes or hollow fibre membranes. The use of modules incorporating hollow fibre membranes is desirable in view of their easy fabrication and readiness for miniaturisation. Filtration membranes used for these modules selectively separate a plasma component into high molecular weight substances and low molecular weight substances and permit optional selection of fractionation molecular weight according to the desired purpose. For example, in the treatment of autoimmune disease (one of the major uses of apparatus of this invention) the fractionation molecular weight can be chosen at a value of 100,000. Pathogenic substances associated with autoimmune diseases are often present in a form bound to γ-globulin of molecular weight about 160,000 and, therefore, in the treatment of these diseases it is desirable to remove substances having molecular weights greater than 160,000 and to return, for example, albumin having a molecular weight of 67,000 lower than 160,000 (and being useful for the living body) to the patient's body. Thus, by choosing a fractionation molecular weight of 100,000. γ-globulin can be sharply fractionated from albumin. The value of the fractionation molecular weight for the filtration membrane should be chosen according to the molecular weight of the pathogenic substances being treated and, in addition to the example referred to above, a fractionation molecular weight is generally chosen of a value between 100,000 and 200,000 when an immunocompound is a cause of the disease. Any kind of filtration membrane may be used for fractionating or separating plasma components under pressure and, for example, membranes having ultrafiltration capability may generally be used. Accordingly, the particular structure of membrane is generally not important and uniform microporous membranes and asymmetrical membranes are all usable.

Materials for filtration membranes having, for example, the above properties include polyvinyl alcohol (PVA) type polymers, ethylene-vinyl alcohol (EVA) copolymers, cellulose derivatives (e.g. cellulose acetate), polyolefins, polyacrylnitriles, polyamides, polyesters, polysulfones and the like.

Among those mentioned above, PVA type, EVA type, cellulose derivatives and polysulfones have excellent physiological acceptability and are desirable for use.

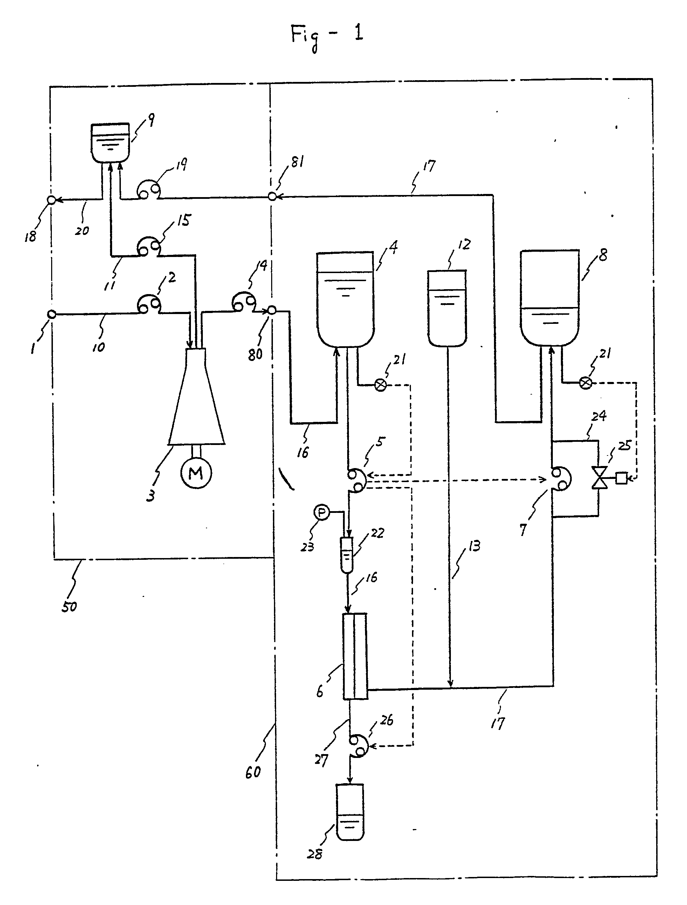

Figure 1 is a flow diagram of a system comprising an apparatus 60 according to an embodiment of the present invention connected to a continuous operation type centrifugal separator 50, and a description of the arrangement thereof is set out below following the flow of blood through the system.

Blood is first fed to the bowl 3 of the centrifugal separator 50, if required, by a pump 2 such as a roller (peristaltic) pump, from a blood inlet 1 (that can be connected to any of the generally used blood letting instruments, such as a shunt and syringe, or to a blood reservoir) through a blood inlet circuit 10, where separation into a corpuscular fraction and plasma takes place. The plasma component separated by the centrifugal separator 50 is continuously fed into the plasma treatment apparatus 60 of this invention. In particular, the plasma component separated by the centrifugal separator is introduced into a plasma feed circuit 16 through a plasma inlet port 80 by a roller pump 14 incorporated into the centrifugal separator 50. The plasma is retained in a plasma reservoir bag 4 connected to circuit 16. Means 21 for sensing the fluid level within reservoir bag 4 is provided. When the quantity of plasma component collected exceeds a predetermined value, a plasma feed pump 5, which is a roller pump controlled in association with the level sensing means 21, operates to remove plasma fraction from the bag 4 and deliver it to a filtration membrane module 6. A drip chamber 22 having a pressure gauge 23 connected thereto is provided in the plasma feed circuit 16 to monitor abnormal pressure caused by clogging of the filtration membrane module 6 or other trouble. When connecting the plasma treatment apparatus 60 of this invention to a centrifugal separator 50, the flow quantity fed by the plasma feed pump 5 may be selected to be slightly larger than that fed by the roller pump 14 which discharges plasma fraction from the centrifugal separator, so as to provide rapid treatment of the plasma fraction collecting in the plasma reservoir bag 4 and minimisation of the quantity of blood in extracorporeal circulation. The plasma fraction fed to the membrane module 6 is separated thereby into high molecular weight substances and low molecular weight substances containing albumin. A purified plasma fraction comprising low molecular weight substances separated by the filtration membrane module 6 is fed to a purified plasma reservoir vessel 8 in a purified plasma return circuit 17 by a purified plasma delivery pump 7 controlled in association with the plasma feed pump 5. The purified plasma fraction from the purified plasma reservoir vessel 8 is fed through a purified plasma outlet port 81 to a mixing device 9 by a roller pump 19 incorporated in the centrifugal separator 50 and therein mixed with the corpuscular fraction from a corpuscular fraction outlet circuit 11 and a roller pump 15 incorporated in the centrifugal separator 50. Purified blood, as a mixture of purified plasma and corpuscular fraction, passes to a purified blood outlet part 18 (that can be connected to the shunt, instillation set, or the like) provided in a purified blood return circuit 20. When not operating the roller pump 19, the mixing device may be used as a purified plasma reservoir vessel.

On the other hand, the condensed plasma component containing high molecular weight sustances separated by the filtration membrane module 6 is discharged by a condensed plasma discharge pump 26 in a condensed plasma discharge circuit 27 to a waste fluid vessel 28. The fluid flow quantity of the pump 26 is usually set to be equal to or under 1/5th to 1/10th of that of the plasma feed pump 5. The aforesaid discharge pump 26 is controlled in association with the plasma feed pump 5 so that the ratio between the quantity of plasma component fed to the membrane module 6 and that of the condensed plasma component taken therefrom assumes a predetermined value. For example, if the fluid flow quantity of the plasma feed pump 5 is set at 30 cc/min, the flow quantity of the plasma discharge pump 26 may be automatically adjusted to be equal to or less than 1/5th to 1/10th of that of the former, that is, 6 to 3 cc/min or less. The purified plasma delivery pump 7 is also operated in association with the plasma feed pump 5 to control the ratio between the plasma quantity fed to the membrane module 6 and that delivered therefrom. For example, when the flow quantity of the plasma feed pump 5 is set at 30 cc/min, the purified plasma delivery pump 7 may be controlled to deliver fluid in a quantity of 24 to 30 cc/min. When the whole quantity of plasma component is filtered by the filtration membrane module 6, the flow quantity of the plasma feed pump 5 and that of the purified plasma delivery pump 7 are set to be equal to each other and the condensed plasma discharge pump 26 is stopped.

A circuit 13 for supplying a supplementary fluid such as albumin or hydroxyethyl starch (HES) from a supplementary fluid reservoir bag 12 to the purified plasma component to compensate for the condensed plasma component removed by said membrane module 6 is connected to the upstream side of the purified plasma delivery pump 7 in the purified plasma return circuit 17. By setting the flow quantity in the purified plasma delivery pump 7 so as to equalise the quantity of plasma fed to the filtration membrane module 6 to the quantity of purified plasma including purified plasma fraction delivered from said module 6 plus supplementary fluid, the same quantity of supplementary fluid as that of the condensed plasma component discharged by the condensed plasma discharge pump 26 may be supplied to the purified plasma reservoir vessel 8. A by-pass circuit 24 is provided for by-passing the purified plasma delivery pump 7. This by-pass circuit 24 generally serves to deliver supplementary fluid through the by-pass to the purified plasma reservoir vessel 8 when a valve 25 is opened when, for example, all pumps in the plasma treatment apparatus are stopped due to troubles occurring such as clogging in the membrane module 6 and resulting replacement of said module 6. When the trouble or fault has been corrected the valve 25 may be closed and normal operation can be started again. Therefore, fluid transfusion into the human body can be performed without interruption even during the down-time of the apparatus, thereby ensuring a high degree of safety.

The means 21 for sensing the fluid level in the plasma reservoir bag 4 is preferably capable of constantly monitoring the fluid level because of the ease in changing the level of fluid to be set. For fluid level sensing, it is preferred to use a method using a pressure sensor which senses the level by pressure, using a weight sensor, or an ultrasonic sensor which directly senses the fluid level with the application of ultrasonic waves.

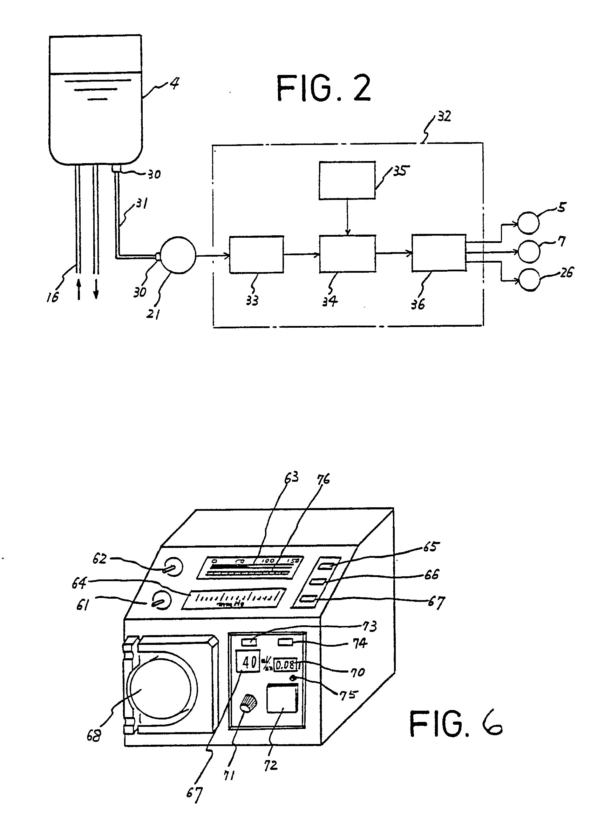

Figure 2 is a representation of an example using a pressure sensor as fluid level sensing means 21, wherein a tube 31 of inner diameter 1 mm or less, usually 0.8 mm, is connected to a pressure sensing hole 30 provided at the lower side of the plasma reservoir bag 4 and a pressure sensor 21 is fixed to the end of tube 31. As a pressure sensor, a diaphragm type pressure converter is conveniently used. When the fluid level changes as the plasma component is poured into the plasma reservoir bag 4, the head pressure exerted on the air contained in the thin tube and the compressed air cause displacement of the diaphragm. Minute displacement of the diaphragm is detected by a strain gauge made of metallic wire or a semi-conductor strain gauge, or the like and detected signals are transmitted to the control device 32 for controlling the operation of each pump. Signals emitted from the pressure sensor are amplified by an amplifier circuit 33 and then transmitted to a comparator circuit 34. In the comparator circuit 34, signals are compared with those corresponding to the fluid level set in the setting circuit 35 and transmitted to a drive circuit 36 for controlling the revolution of the pumps 5, 7 and 26. By means of the fluid level sensing means 21, the quantity of plasma component reserved in the plasma reservoir bag 4 is sensed and, at the same time, the number of revolutions of the plasma feed pump 5 is automatically changed under control performed in association with said sensing means 21, or the fluid level in the plasma reservoir bag 4 can be controlled to be within a predetermined range by automatically turning ON - OFF the switch. For example, when the fluid level falls lower than a predetermined level, an adjustment may be made by retarding or stopping the plasma feed pump 5.

Fluid level sensing means 21 may also be conveniently disposed in the purified plasma reservoir vessel 8. In this case, the valve 25 provided in the circuit 24 by-passing the purified plasma delivery pump may be controlled in association with this fluid level sensing means. That is to say, when the fluid level in the purified plasma reservoir vessel 8 falls lower than a predetermined value, the valve 25 is opened for feeding supplementary fluid into the reservoir vessel 8 and closed with the return of the fluid level to the initial position. Such a control effected by fluid level sensing means in association with the valve constantly keeps the quantity of purified plasma component at a predetermined level in the purified plasma reservoir vessel 8. Therefore, accidental entraining of air into the purified plasma fraction to be returned to the patient's body possibly caused by fluid shortage can be completely prevented.

Means for controlling the fluid quantity in said pumps 5, 7 and 26, conveniently employ a method for electrically controlling the number of revolutions of these pumps. Further, a duplex or triplex type roller pump may also be usable. In this case, an adjustment of the fluid quantity flowing in each circuit is made possible by changing the diameter or corresponding tube.

As a plasma reservoir bag 4, a flexible bag having a capacity of 50 to 3,000 cc, for example, a blood bag, can be used. A flexible bag having a capacity usually ranging from 200 to 3,000 cc is used as a purified plasma reservoir bag 8, however, a vessel having a capacity of 10 to 50 cc may be used when the purified plasma fraction is returned to the patient's body by means of the purified plasma delivery pump. Such a vessel may be, for example, a drip chamber for dialysis.

A mixing device is conveniently used for mixing the purified plasma component with a corpuscular fraction and advantageously comprises a flexible bag or drip chamber. A plasma warmer for warming the plasma fraction cooled during extracorporeal circulation, or a pre-filter for removing material substances contained in the plasma component may additionally be provided at the upstream side of the filtration membrane module 6.

Figures 3 to 5 are diagrammatic flow-diagrams of a system in which a batch type centrifugal separator 50 and embodiments of a plasma treatment apparatus 60 according to the present invention are combined. A difference between the batch type and the continuous operation type of separator is such that, in the batch type separator, sterilised air flows into or out from the bowl 3 during the process of blood letting or blood returning. Namely, sterilised air which enters the centrifugal bowl 3 at the initial stage is driven out toward the plasma reservoir bag 4 by blood introduced into the bowl 3 at the time of blood letting but, on the other hand, at the time of returning blood to the patient, the sterilised air flowing into the bowl 3 drives out the corpuscular fraction collected in the bowl. Accordingly, attention must be paid for preventing the plasma fraction containing high molecular weight substances from being mixed with sterilised air flowing into the bowl when returning blood to the patient. Whereas the continuous operation type centrifugal separator uses a "two-arm" method in which different syringes are separately used for blood letting and returning, a batch type separator uses either a "single-arm" method in which a mixture of purified plasma with corpuscular fraction may be returned to the patient's body through the blood inlet part as well as a two-arm method using different syringes separately for blood letting and blood (or plasma) returning. Figure 3 shows an example of a system employing the two-arm method using the plasma treatment apparatus 60 of the present invention combined with a batch type centrifugal separator 50, wherein, during blood letting, blood is introduced at the blood inlet 1 and flows through the blood inlet circuit 10 into the bowl 3 of the centrifugal separator 50. Then, sterilized air in the centrifugal bowl 3 is driven out by the pressure of blood fed by pump 2, and conveyed, prior to the advance of the separated plasma fraction, to the plasma reservoir bag 4 through the plasma inlet port 80 and the plasma feed circuit 16.

On the other hand, a separated corpuscular fraction collects in the centrifuge bowl 3. When a corpuscular fraction detector 43 detects that the corpuscular fraction collecting in the centrifugal bowl overflows said bowl and starts flowing into the plasma feeding circuit, the apparatus is stopped and blood letting suspended and blood returning started. During blood letting, the separated plasma fraction is treated by the plasma treatment apparatus 60 of this invention. That is, the plasma fraction collected in the plasma reservoir bag 4 is passed to the filtration membrane module 6 in similar manner as in the case of the continuous operation type separator apparatus described above and thereby separated into high molecular weight substances and low molecular weight substances. The purified plasma fraction containing low molecular weight substances separated by the membrane module 6 is delivered to the purified plasma reservoir vessel 8 by the plasma fraction delivery pump 7 controlled in association with the plasma feed pump 5. The purified plasma fraction collected in the purified plasma reservoir vessel 8 may be continuously returned to the patient's body from the purified plasma outlet port 81 provided in the purified plasma return circuit 17. In the course of blood returning, the roller pump 2 for pumping blood to the centrifuge bowl 3 is rotated in the counter direction, so that the cor-

puscular fraction collecting in the bowl 3 is sucked into the blood inlet circuit 10 and afterward returned to the patient's body from the blood inlet 1 through said circuit. Such blood returning is performed from the centrifugal separator 50. When the corpuscular fraction in the bowl 3 is removed, a reduced pressure is generated in the bowl 3 and sterilised air filling an upper space of the plasma reservoir bag 4 is sucked back into the bowl 3 through the plasma feeding circuit 16. When the corpuscular fraction is completely replaced by sterilised air sucked into the bowl 3 and the sterilised air is further sucked back into the blood inlet circuit 10 by the roller pump 2 to flow into the blood inlet circuit 10, a bubble detector 42 provided in circuit 10 operates to stop the pump 2 and stop the blood returning process. The above two processes of blood letting and blood returning may be regarded as one cycle to be repeated as many times as required. In the batch type centrifugal separator 50, when sterilised air in the plasma reservoir bag 4 is sucked into the centrifugal bowl 3, care must be taken to prevent the plasma fraction from flowing into the bowl 3 together with the sterilised air. In this embodiment, the plasma inlet port of the plasma reservoir bag 4 is designed to prevent the plasma fraction from flowing back into the bowl 3. In particular, the plasma inlet port for the plasma reservoir bag 4 is disposed such that it opens at a position higher than that of the fluid level of the plasma fraction collecting in the plasma reservoir bag 4, that is, a position in the upper space in the bag 4, and a plasma outlet is disposed at a lower position in the bag 4. The plasma inlet for the plasma reservoir bag 4 may assume a configuration extending from above and opening into the upper space in the bag 4 or it may comprise an independent plasma inlet conduit in the bag. A suitable plasma reservoir bag 4 is a commercially available blood bag which can be used after having a blood inlet port thereof modified to form the plasma inlet port.

When using a system comprising a batch type centrifugal separator connected to a plasma treatment apparatus according to the present invention in a "two-arm" method, it is preferable to provide a valve 82 controlled by the fluid level sensing means 21 in the purified plasma reservoir vessel 8 in the purified plasma return circuit 17. In this arrangement, the level sensing means 21 is not required to operate in association with a valve 25 provided in the by-pass circuit 24 for the purified plasma delivery pump 7. Co-operative operation of the level sensing means 21 with the valve 82 enables prevention of accidental mixing of air with blood in the patient's body caused by fluid shortage in the purified plasma reservoir vessel.

As shown in Figure 4, the purified plasma outlet port 81 of the purified plasma return circuit 17 and the blood inlet port 1 of the blood inlet circuit 10 may be connected to a Y-shaped connector 45 connected to the shunt or the like to allow the system to be employed in the single-arm method.

In this method the corpuscular fraction and purified plasma are mixed together with the Y-shaped connector 45 and returned to the patient's body. In this case, it is necessary to provide a changeover valve 46 in the circuit which connects the purified plasma outlet port 81 of the purified plasma return circuit 17 with the Y-shaped connector 45 so that the purified plasma fraction is prevented from entering the centrifugal separator bowl 3 together with blood during blood letting. The changeover valve 46 operates in association with the corpuscular fraction detector 43 and bubble detector 42 and can automatically be controlled so as to be closed and opened at the time of blood letting the blood returning, respectively. Further, when this changeover valve 46 is controlled in association with the fluid level sensing means 21 in the purified plasma reservoir vessel 8, accidental entry of air into the patient's body, e.g. because of shortage of fluid in the reservoir vessel, can be prevented.

The changeover valve 46 is preferably a pinch valve which directly clamps or opens the circuit path so that blood does not directly contact the valve parts.

Figure 5 is a representation of another example for a two-arm method of mixing the corpuscular fraction with the purified plasma component and then returning them to the patient's body. In this embodiment, the blood inlet circuit 10 is connected to the purified plasma reservoir vessel 8 by a corpuscular fraction feeding circuit 44. The corpuscular fraction feeding circuit 44 and blood feeding circuit 10 are capable of being changed over. Figure 5 shows that fluid passage is so designed as to be changed over by two changeover valves 40 and 41 provided in respective circuits. The purified plasma reservoir vessel 8 (corresponding to the blood reservoir vessel in the batch type centrifugal separator), circuits 10, 20 and 44, and changeover valves 40 and 41 are associated with the centrifugal separator 50 in the two-arm method type. Therefore, when the purified plasma returning circuit 17 is connected to a corpuscular fraction reservoir vessel of the centrifugal separator, the corpuscular fraction reservoir vessel serves also as a purified plasma reservoir vessel. An apparatus thus arranged operates as follows:

First, a changeover valve 41 closes and the other changeover valve 40 opens for blood letting so that blood passes to the centrifugal separator bowl 3 in which blood is separated into a corpuscular fraction and plasma, and sterilised air driven out from the bowl 3 is reserved in the plasma reservoir bag 4. The separated plasma component is treated in the filtration membrane module 6 and the plasma component thereby freed of high molecular weight substances is conveyed to the purified plasma reservoir vessel 8. The purified plasma fraction is continuously returned to the patient's body. When the corpuscular fraction detector 43 detects that a corpuscular fraction starts to flow into the plasma feeding circuit 16, the blood letting process is terminated and a mixing of purified plasma with the corpuscular fraction then starts. In the mixing process, the changeover valve 40 closes, the other valve 41 opens and the pump 2 is rotated in the counter direction. Corpuscular fraction from the centrifugal bowl 3 is delivered to the purified plasma reservoir vessel 8 through the corpuscular fraction feeding circuit 44 where the corpuscular fraction is mixed with the purified plasma component. When the corpuscular fraction in the bowl 3 has been replaced by sterilised air and the bubble detector 42 detects that sterilised air flows from the bowl 3, the mixing process is terminated and blood letting recommenced.

The process of returning purified blood containing both a corpuscular fraction and a purified plasma component from the purified plasma reservoir vessel 8 to the patient's body is conducted in parallel with blood letting as a second process at the same time. In the purified blood return circuit 20, there may be provided a changeover valve 82 controlled in association with fluid level sensing means 21 provided in said reservoir vessel 8 to prevent accidental entry of air into the patient's body as a result of fluid shortage in the purified plasma reservoir vessel 8.

For simplification of the description, parts in Figures 3 to 5 identical to those in Figure 1 are herein indicated by the same reference numerals as those in Figure 1.

Figure 6 is a perspective view of a monitoring unit comprising a plasma treatment apparatus according to an embodiment of the present invention. Figure 6 will be described with reference also to the systems represented in the other Figures. A plasma feeding circuit 16 and purified plasma feeding circuit 17 may be connected to a duplex pump 68 (in place of pumps 5 and 7) provided in the front part of the monitoring unit. The pipe 31 (Figure 2) for measuring the head pressure in the plasma reservoir bag 4 with the pressure sensor 21 is connected to a connector 61, and another pipe for measuring the pressure in the drip chamber 22 in the plasma feeding circuit with the pressure gauge 23 is connected to another connector 62. Reference numeral 63 represents a level indicator for indicating the fluid level in the plasma reservoir bag 4 and which indicates the head pressure in terms of a bar graph. A switch designated as 76 is intended to permit optional setting of the fluid level of plasma. Numeral 64 indicates a pressure gauge for indicating the pressure on the inlet side of the filtration membrane module 6 and 71 indicates a knob for setting the flow quantity in the plasma feed pump 5. The total quantity of plasma fraction fed to the membrane module 6 by pump 5 is indicated by the integrating indicator 70. Reference numerals 72 and 73 indicate a main switch and a changeover switch respectively, which effect changeover between an automatic function and a manual function. During automatic operation, a lamp 74 is illuminated and the pump is rotated when the level of plasma fraction in the plasma reservoir bag 4 exceeds a preset value. When the plasma fraction level falls below a preset value, lamp 74 is turned off and the pump stops rotating. The numeral 75 represents an integration resetting button. A lamp which will light to indicate any abnormality is provided for said monitor. Numeral 65 indicates a pressure alarm lamp actuated when the pressure on the inlet side of the membrane module 6 passes upper or lower alarm limits. 66 is a lamp lit when the cover of the pump 68 is opened and 67 is a lamp lit when the amount of plasma in the plasma reservoir bag 4 exceeds an allowed value.

The control systems of the plasma treatment apparatus are all conveniently contained in the above described monitor unit. Therefore, the centrifugal separator 50 and the apparatus 60 of the invention can be fully controlled separately from each other when connected together to operate as a systematised unit.

As has been described above, apparatus according to the present invention can operate as a part of a systematised unit with the provision of a plasma reservoir bag 4 and purified plasma reservoir bag 8 as well as by having fluid level sensing means in the plasma reservoir bag 4, a plasma feed pump 5, and a purified plasma delivery pump 7 which are controlled in association with each other when connected to any type of centrifugal separator currently used and which differ from each other in their method of performance.

The use of such a system as above described ensures effective and safe removal of harmful substances from blood without damage to and loss of erythrocytes and thrombocytes or loss of desired serum proteins.

**Claims**

1. An apparatus for the treatment of plasma of a patient, which is connectable to a centrifugal separator (50) disposed in an extracorporeal circuit so as to separate a plasma component separated from blood by the centrifugal separator into high molecular weight substances and low molecular weight substances containing albumin by means of a filtration membrane module (6) and to return purified plasma freed of high molecular weight substances to the body of the patient, comprising: a plasma feeding circuit (16) for connecting the centrifugal separator (50) disposed in said extracorporeal circuit with the filtration membrane module (6); a plasma reservoir (4) in said circuit; a plasma feed pump (5); a circuit (17) for returning purified plasma containing albumin and freed of high molecular weight substances by said filtration membrane module (6) to the patient's body; a purified plasma reservoir vessel (8) in said circuit (17); a purified plasma delivery pump (7) in the circuit (17) for returning purified plasma to the patient's body, in which pump the fluid flow ratio between plasma introduced to the filtration member (6) and purified plasma delivered therefrom is at a predetermined value and controlled in association with said plasma feed pump (5); and a condensed plasma discharge pump (26) the flow rate of which is set relative to that of the plasma feed pump (5) such that the fluid flow ratio between the plasma component delivered to the filtration member module (6) and high molecular weight substances discharged therefrom to a condensed plasma discharge circuit (27) from the filtration membrane module (6) is at a predetermined value; characterised in that the apparatus includes means (21) for sensing the fluid level in said plasma reservoir (4) whereby the fluid flow rate in the plasma feed pump (5) can be adjusted and controlled in association with said fluid level sensing means (21) so that the fluid level is within a predetermined range; a supplementary fluid circuit (13) connected to the upstream side of the purified plasma delivery pump (7); and a circuit (24) for bypassing the purified plasma delivery pump (7) when flow of purified plasma is interrupted, causing the purified plasma delivery pump (7) to be stopped.

2. An apparatus for plasma treatment as claimed in claim 1 in which the fluid level sensing means (21) is a pressure sensor which senses the head pressure of plasma in the plasma reservoir (4).

3. An apparatus for plasma treatment as claimed in either of claims 1 and 2 connected to a centrifugal separator (50) which is a continuous operation type centrifugal separator.

4. An apparatus for plasma treatment as claimed in claim 3, in which a fluid level sensing means (21) is provided in the purified plasma reservoir vessel (8) and a changeover valve (25) which opens or closes while controlled in association with said purified plasma reservoir vessel level sensing means (21) is provided in the circuit (24) by-passing the purified plasma delivery pump (7).

5. An apparatus for plasma treatment as claimed in either of claims 1 and 2 connected to a centrifugal separator (50) which is a batch type centrifugal separator.

6. An apparatus for plasma treatment as claimed in claim 5, in which a plasma inlet port opening into the upper space of plasma reservoir (4) is provided.

7. An apparatus for plasma treatment as claimed in either of claims 5 and 6, in which the purified plasma reservoir vessel (8) is connected by a corpuscule feed circuit (44) to a circuit (10) for introducing blood into the centrifugal separator (50), and a changeover valve (41) for closing said corpuscule feed circuit (44) during blood letting and blood returning is provided in said corpuscule feed circuit, and another changeover valve (40) for closing said blood inlet circuit (10) during blood mixing is provided in said blood inlet circuit (10).

8. An apparatus for plasma treatment as claimed in claim 6, in which the purified plasma return circuit (17) and circuit (10) for introducing blood into the centrifugal separator (50) are connected to a Y-shaped connector (45), and a changeover valve (46) for closing said return circuits (17) during blood letting is provided in said purified plasma return circuit (17).

**Patentansprüche**

1. Gerät für die Behandlung von Plasma eines Patienten, das mit einem in einem Kreis außerhalb des Körpers angeordneten Zentrifugalseparator (50) zu verbinden ist, um eine durch den Zentrifugalseparator vom Blut separierte Plasmakomponente in Stoffe mit hohem Molekulargewicht und Albumin enthaltende Stoffe mit niedrigem Molekulargewicht mittels eines Filtrationsmembranmoduls (6) zu trennen und das geklärte Plasma, das von den Stoffen mit hohem Molekulargewicht befreit ist, zum Körper des Patienten zurückzuführen, aufweisend: einen Plasmazuführkreis (16) zum Verbinden des in dem Kreis außerhalb des Körpers angeordneten Zentrifugalseparators (50) mit dem Filtrationsmembranmodul (6); ein Plasmareservoir (4) in diesem Kreis; eine Plasmazuführpumpe (5); einen Kreis (17) zum Zurückführen von geklärtem Plasma, das Albumin enthält und durch den Filtrationsmembranmodul (6) von Stoffen mit hohem Molekulargewicht befreit ist, zum Körper des Patienten; einen Speicherbehälter (8) für geklärtes Plasma in diesem Kreis (17); eine Speisepumpe (7) für geklärtes Plasma in dem Kreis (17) zum Zurückführen von geklärtem Plasma zum Körper des Patienten, in der das Fluidflußverhältnis zwischen in das Filtrationselement (6) eingeführtem Plasma und davon geliefertem geklärtem Plasma auf einem vorgegebenen Wert liegt und im Zusammenhang mit der Plasmazuführpumpe (5) gesteuert wird; und eine Abführpumpe (26) für kondensiertes Plasma, deren Flußrate relativ zu der der Plasmazuführpumpe (5) so eingestellt wird, daß das Fluidflußverhältnis zwischen der zu dem Filtrationselementmodul (6) gelieferten Plasmakomponente und Stoffen mit hohem Molekulargewicht, die davon zu einem Abführkreis (27) für kondensiertes Plasma von dem Filtrationsmembranmodul (6) abgeführt werden, auf einem vorgegebenen Wert liegt; dadurch gekennzeichnet, daß das Gerät enthält: eine Einrichtung (21) zum Abfühlen des Fluidpegels in dem Plasmareservoir (4), wodurch die Fluidflußrate in der Plasmazuführpumpe (5) eingestellt und im Zusammenhang mit der Fluidpegel-Abfühleinrichtung (21) gesteuert werden kann, so daß der Fluidpegel in einem vorgegebenen Bereich liegt; einen Ergänzungsfluidkreis (13), der mit der in Flußrichtung oberen Seite der Speisepumpe (7) für geklärtes Plasma verbunden ist; und einen Kreis (24) zum Umgehen der Speisepumpe (7) für geklärtes Plasma, wenn der Fluß von geklärtem Plasma unterbrochen wird, was dazu führt, daß die Speisepumpe (7) für geklärtes Plasma angehalten wird.

2. Gerät für die Plasmabehandlung nach Anspruch 1, in dem die Fluidpegel-Abfühleinrichtung (21) ein Drucksensor ist, der den Kopfdruck von Plasma in dem Plasmareservoir (4) abfühlt.

3. Gerät für die Plasmabehandlung nach Anspruch 1 oder 2, das mit einem Zentrifugalseparator (50) verbunden ist, der ein Zentrifugalseparator für kontinuierlichen Betrieb ist.

4. Gerät für die Plasmabehandlung nach Anspruch 3, in dem eine Fluidpegel-Abfühleinrichtung (21) in dem Speicherbehälter (8) für geklärtes Plasma vorgesehen ist und ein Umschaltventil (25), das öffnet oder schließt, während es im Zusammenhang mit der Pegel-Abfühleinrichtung (21) in dem Speicherbehälter für geklärtes Plasma gesteuert wird, in dem Kreis (24) vorgesehen ist, der die Speisepumpe (7) für geklärtes Plasma umgeht.

5. Gerät für die Plasmabehandlung nach Anspruch 1 oder 2, das mit einem Zentrifugalseparator (50) verbunden ist, der ein Zentrifugalseparator für diskontinuierlichen Betrieb ist.

6. Gerät für die Plasmabehandlung nach Anspruch 5, in dem eine Plasmaeinlaßöffnung vorgesehen ist, die sich in den oberen Raum des Plasmareservoirs (4) öffnet.

7. Gerät für die Plasmabehandlung nach Anspruch 5 oder 6, in dem der Speicherbehälter (8) für geklärtes Plasma durch einen Korpuskel-Zuführkreis (44) mit einem Kreis (10) für die Einführung von Blut in den Zentrifugalseparator (50) verbunden ist und ein Umschaltventil (41) zum Schließen des Korpuskel-Zuführkreises (44) während des Einlassens von Blut und des Rückführens von Blut in dem Korpuskel-Zuführkreis vorgesehen ist und ein weiteres Umschaltventil (40) zum Schließen des Blut-Einlaßkreises (10) während des Mischens von Blut in dem Blut-Einlaßkreis (10) vorgesehen ist.

8. Gerät für die Plasmabehandlung nach Anspruch 6, in dem der Rückführkreis (17) für geklärtes Plasma und der Kreis (10) zum Einführen von Blut in den Zentrifugalseparator (50) mit einem Y-förmigen Verbinder (45) verbunden sind und ein Umschaltventil (46) zum Schließen des Rückführkreises (17) während des Einlassens von Blut in dem Rückführkreis (17) für geklärtes Plasma vorgesehen ist.

**Revendications**

1. Dispositif de traitement de plasma d'un patient, qui peut être relié à un séparateur centrifuge (5) disposé dans un circuit extracorporel de manière à séparer un composant de plasma séparé du sang par le séparateur centrifuge en des substances de poids moléculaire élevé et des substances de faible poids moléculaire contenant de l'albumine au moyen d'un module à membrane de filtration (6) et de renvoyer le plasma purifié ne contenant plus de substances de poids moléculaire élevé dans le corps du patient, comprenant: un circuit d'alimentation de plasma (16) pour relier le séparateur centrifuge (50) disposé dans ledit circuit extracorporel au module à membrane de filtration (6), un réservoir à plasma (4) dans ledit circuit; une pompe d'alimentation de plasma (5); un circuit (17) pour renvoyer le plasma purifié contenant de l'albumine et exempt de substances de poids moléculaire élevé par ledit module à membrane de filtration (6) vers le corps du patient; un réceptacle formant réservoir de plasma purifié (8) dans ledit circuit (17); une pompe d'alimentation de plasma purifié (7) dans le circuit (17) pour renvoyer le plasma purifié dans le corps du patient, pompe dans laquelle le rapport des débits entre le plasma introduit dans l'organe de filtration (6) et le plasma purifié fourni par celui-ci est à une valeur prédéterminée et commandée en association avec ladite pompe d'alimentation de plasma (5); et une pompe de décharge de plasma condensé (26) dont le débit est réglé par rapport à celui de la pompe d'alimentation de plasma (5) de manière que le rapport des débits entre le composant de plasma fourni par le module à organe de filtration (6) et les substances de poids moléculaire élevé qui en sont déchargées vers un circuit de décharge de plasma condensé (27) provenant du module à membrane de filtration (6) est à une valeur prédéterminée; caractérisé en ce que le dispositif comprend un moyen (21) pour détecter le niveau du fluide dans ledit réservoir à plasma (4), le débit de fluide dans la pompe d'alimentation de plasma (5) pouvant être ajusté et commandé en association avec ledit moyen de détection de niveau de fluide (21) de manière que le niveau du fluide soit dans une plage prédéterminée; un circuit de fluide supplémentaire (13) relié au côté amont de la pompe d'alimentation de plasma purifié (7); et un circuit (24) pour passer en dérivation de la pompe d'alimentation de plasma purifié (7) quand l'écoulement du plasma purifié est interrompu, provoquant l'arrêt de la pompe d'alimentation de plasma purifié (7).

2. Dispositif de traitement de plasma selon la revendication 1, dans lequel le moyen de détection de niveau de fluide (21) est un détecteur de pression qui détecte la pression du plasma dans le réservoir à plasma (4).

3. Dispositif de traitement de plasma selon la revendication 1 ou 2, relié à un séparateur centrifuge (50) qui est un séparateur centrifuge du type à fonctionnement continu.

4. Dispositif de traitement de plasma selon la revendication 3, dans lequel un moyen de détection de niveau de fluide (21) est prévu dans le réceptacle formant réservoir à plasma purifié (8) et une soupape de commutation (25) qui s'ouvre et se ferme quand elle est commandée en association avec ledit moyen de détection de niveau (21) du réceptacle formant réservoir à plasma purifié est prévu dans le circuit (24) qui est en dérivation de la pompe d'alimentation de plasma purifié (7).

5. Dispositif de traitement de plasma selon la revendication 1 ou 2, relié à un séparateur centrifuge (50) qui est un séparateur centrifuge du type à lots.

6. Dispositif de traitement de plasma selon la revendication 5, dans lequel est prévue une ouverture d'entrée de plasma débouchant dans l'espace supérieur du réservoir à plasma (4).

7. Dispositif de traitement de plasma selon la revendication 5 ou 6, dans lequel le réceptacle formant réservoir à plasma purifié (8) est relié par un circuit d'alimentation de corpuscules (44) à un circuit (40) destiné à l'introduction du sang dans le séparateur centrifuge (50), et une soupape de commutation (41) pour fermer ledit circuit d'alimentation de corpuscules (44) pendant l'admission du sang et le renvoi du sang est prévue dans ledit circuit d'alimentation de corpuscules, et une autre soupape de commutation (40) pour fermer ledit circuit d'entrée de sang (10) pendant le mélange du sang est prévue dans ledit circuit d'entrée de sang (10).

8. Dispositif de traitement de plasma selon la revendication 6, dans lequel le circuit de renvoi de plasma purifié (17) et le circuit (10) pour introduire le sang dans le séparateur centrifuge (50) sont reliés à un connecteur en forme de Y (45), et une soupape de commutation (46) pour fermer lesdits circuits de renvoi (17) pendant l'admission du sang est prévue dans ledit circuit de renvoi de plasma purifié (17).

Fig - 1

# FIG. 2

*4*

*32*

*35*

*30*

*31*

*30*

*21*

*33*

*34*

*36*

*16*

*5*

*7*

*26*

# FIG. 6

*63*  *76*

*62*

*64*

*61*

*65*

*66*

*67*

*73*

*74*

*70*

*75*

*68*

40 ml/43    0.08

*67*

*71*

*72*

Fig - 3

Fig - 4

3

Fig - 5